# EUROPEAN PATENT APPLICATION

(11) **EP 1 738 704 A1**
(43) Date of publication of application: **03.01.2007**
(21) Application number: 04821908.3
(22) Date of filing: 12.11.2004
(51) Int. Cl.: A61B 18/00

(54) **ULTRASONIC TREATMENT TOOL**

(30) Priority: 19.04.2004 JP 2004122868
(71) Applicant: Olympus Corporation, Tokyo 151-0072 (JP)
(72) Inventor: YAMADA, Norihiro, 1920045 (JP); SUZUKI, Keita, 1850035 (JP); NAKAMURA, Takeaki, 1920065 (JP)
(74) Representative: von Hellfeld, Axel
(86) International application number: PCT/JP2004/016878
(87) International publication number: WO 2005/102198

(57) **Abstract**

An ultrasonic treatment apparatus (1) for giving ultrasonic treatment to a desired part in a body cavity with an ultrasonic transducer unit (2) projected from a distal end of an endoscope which is introduced into the body cavity, includes a supporting member (3) which rotatably supports the ultrasonic transducer unit (2) at an ultrasonic vibration node, and an operation wire (7) which operates the ultrasonic transducer unit (2). The supporting member (3) is attached to one end of a sheath (5) provided with an operation unit (6) at the other end. The ultrasonic treatment apparatus (1) is provided, having excellent usability, with easiness in insertion and removal of an ultrasonic generating unit (2) into and from a channel (15b; 16a; 21a) of an endoscope. The ultrasonic treatment apparatus (1) allows suppressing an energy loss of the ultrasonic transducer unit (2) as little as possible since the ultrasonic transducer unit (2) is supported at an ultrasonic vibration node.

## Description

### TECHNICAL FIELD

The present invention relates to an ultrasonic treatment apparatus.

### BACKGROUND ART

Conventionally, an ultrasonic treatment apparatus has been used for giving ultrasonic treatment to a desired part such as a diseased part, being inserted into a channel via a venting cap of an endoscope with its distal end projected from the endoscope (see Patent Document 1, for example).

Patent Document 1: Japanese Patent Application Laid-Open No. H02-191423

### DISCLOSURE OF INVENTION

### PROBLEM TO BE SOLVED BY THE INVENTION

A conventional endoscope, as disclosed in Patent Document 1, includes a channel formed inside a linear insert part to be inserted into a body cavity. The endoscope further includes a venting cap attached at a slant to a proximal part of the linear insert part for inserting an ultrasonic treatment apparatus into the channel, having a structure called "K branch" where the venting cap is inclined to the insert part, i.e., the channel. Besides, there is another endoscope having an inclined channel at the distal end of the insert part which is inserted into a body cavity (hereinafter referred to as an angled endoscope). Consequently, an ultrasonic treatment apparatus provided with an ultrasonic generating unit at the distal end thereof has difficulty in insertion and removal of the ultrasonic generating unit into and from the channel due to an interference with the inner wall of the channel at the K branch of the venting cap or the distal end part of the angled endoscope. Specifically, the difficulty becomes worse when the length of the ultrasonic generating unit is more than that of the venting cap.

In view of the foregoing, an object of the present invention is to provide an ultrasonic treatment apparatus which has excellent usability, with easiness in insertion and removal of an ultrasonic generating unit into and from a channel of an endoscope.

### MEANS FOR SOLVING PROBLEM

To solve the above problems and to achieve the object, an ultrasonic treatment apparatus according to claim 1 is for giving an ultrasonic treatment to a desired part in a body cavity with an ultrasonic generating unit projected from a distal end of an endoscope which is introduced-into the body cavity, and includes a supporting member which rotatably supports the ultrasonic generating unit at an ultrasonic vibration node; and an operation wire which operates to rotate the ultrasonic generating unit.

According to the ultrasonic treatment apparatus set forth as claim 1, the ultrasonic generating unit is easily inserted into and removed from the channel of the endoscope, resulting in excellent usability.

In the ultrasonic treatment apparatus as set forth in claim 2, the supporting member is attached to one end of a sheath which is provided with an operation unit at the other end.

In the ultrasonic treatment apparatus as set forth in claim 3, the operation unit is provided with an operation dial for operating the operation wire.

In the ultrasonic treatment apparatus as set forth in claim 4, the operation wire is laid inside the sheath, both ends being connected to an outside of the ultrasonic generating unit at the ultrasonic vibration node and a middle portion being wound around a winding member provided with the operation dial.

In the ultrasonic treatment apparatus as set forth in claim 5, the operation dial includes a fixing unit which fixes an operation position and holds the ultrasonic generating unit at a desired position.

### EFFECT OF THE INVENTION

In the ultrasonic treatment apparatus according to the present invention, the ultrasonic generating unit rotates in accordance with a shape of the channel when inserted into the channel, thereby allowing easy insertion and removal of the ultrasonic generating unit into and from the channel, resulting in excellent usability.

### BRIEF DESCRIPTION OF DRAWINGS

FIG. 1 is a front view of a partial cross-section of a schematic structure of an ultrasonic treatment apparatus;
FIG. 2 is a perspective view showing an ultrasonic generating unit, a supporting member, and a sheath which constitute the ultrasonic treatment apparatus shown in FIG. 1;
FIG. 3 is a cross-sectional view of the ultrasonic generating unit, the supporting member, and the sheath , taken in a lengthwise direction;
FIG. 4A is a cross-sectional view of a first modification in which a supporting cover supports an ultrasonic transducer unit;
FIG. 4B is a cross-sectional view of a second modification in which the supporting cover supports the ultrasonic transducer unit;
FIG. 5 is a plan view of FIG. 2;
FIG. 6 is a perspective view of an operation unit of the ultrasonic treatment apparatus shown in FIG. 1;
FIG. 7 is a perspective view of the ultrasonic generating unit shown in FIG. 2 in a rotational state;
FIG. 8 is a front view of one example of an endoscope into which the ultrasonic treatment apparatus shown in FIG. 1 is inserted;
FIG. 9 is a cross-sectional view of the endoscope shown in FIG. 8 with the ultrasonic treatment apparatus shown in FIG. 1 inserted through a venting cap of the endoscope;
FIG. 10 is a cross-sectional view of the endoscope shown in FIG. 8 with a treatment unit of the ultrasonic treatment apparatus shown in FIG. 1 projected from a distal end of the endoscope;
FIG. 11 is a cross-sectional view of a relevant part of an oblique-viewing endoscope with the ultrasonic treatment apparatus shown in FIG. 1 inserted into a channel of the oblique-viewing endoscope; and
FIG. 12 is a cross-sectional view of a relevant part of the oblique-viewing endoscope shown in FIG. 11 with the treatment unit of the ultrasonic treatment apparatus shown in FIG. 1 projected from the distal end of the channel of the oblique-viewing endoscope.

### EXPLANATIONS OF LETTERS OR NUMERALS

- 11: Ultrasonic treatment apparatus
- 2: Ultrasonic transducer unit
- 2a: Horn
- 2b: Piezoelectric device
- 2c: Treatment unit
- 2d: Cylinder
- 2e: Wire groove
- 2f: Protrusion
- 3: Supporting cover
- 3a: Cylindrical portion
- 3b: Supporting arm
- 3c: Supporting pin
- 4: Wiring
- 5: Sheath
- 6: Operation unit
- 6a: Output port
- 6b: Operation dial
- 6c: Pulley
- 6d: Hand screw
- 7: Operation wire
- 8: Slack remover
- 7a: Wire pin.
- 10: Endoscope
- 11: Operation unit
- 16: Insert part
- 12: Eyepiece unit
- 13: Main body of operation unit
- 14: Grip unit
- 15: Main body of joint
- 15a: Venting cap
- 15b: Channel
- 16a: Channel
- 16b: Curved frame
- 16c: Observation window
- 16d: Objective lens
- 16e: Image guide
- 20: Oblique-viewing endoscope
- 21a, 21b: Channels
- 21c: Observation window
- 21d: Observation optical system
- 21e: Image pick-up device

### BEST MODE(S) FOR CARRYING OUT THE INVENTION

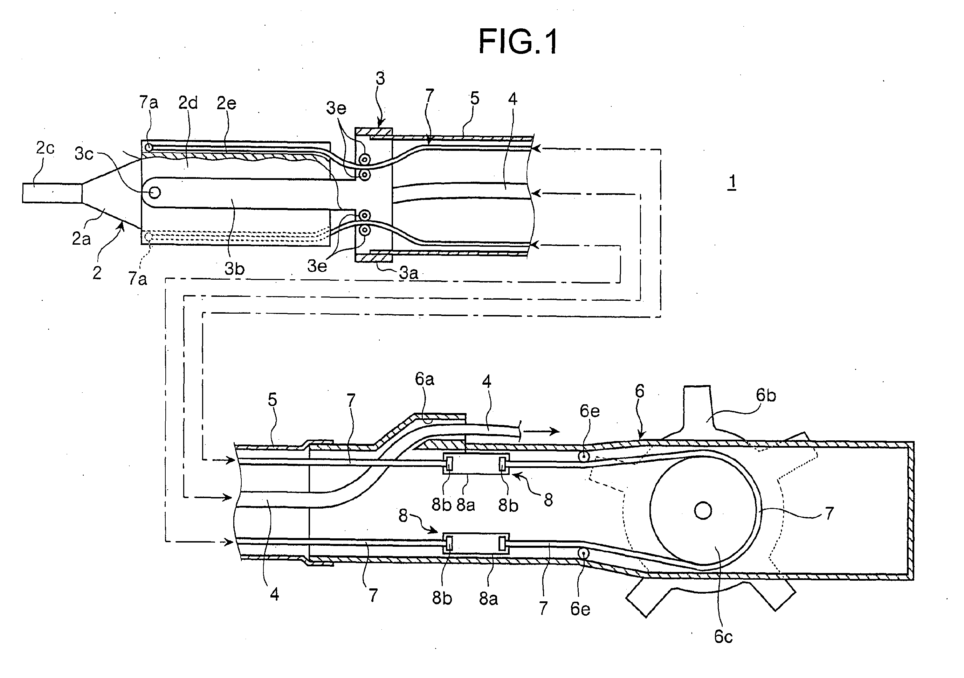

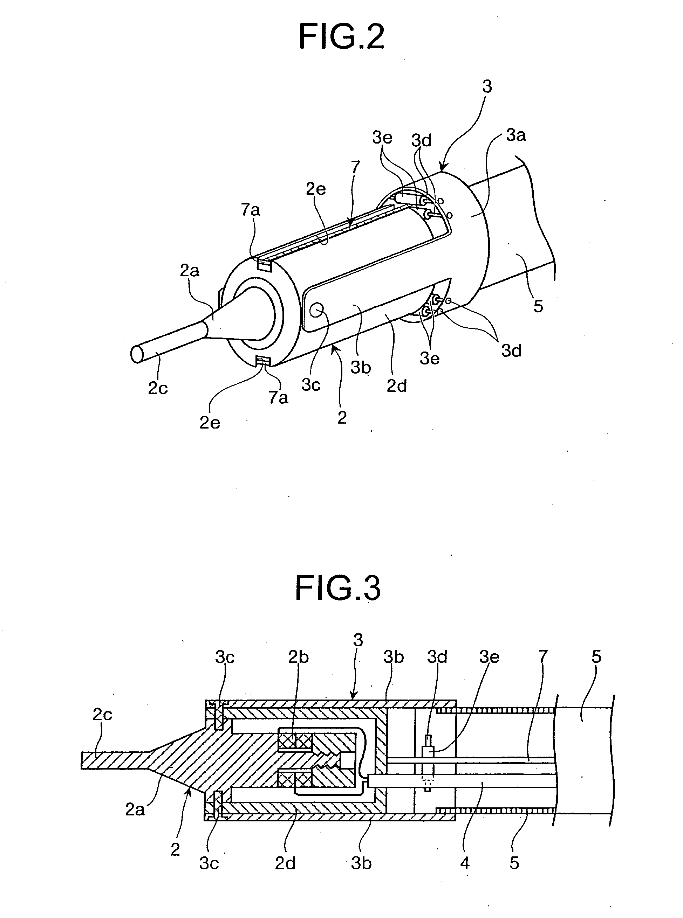

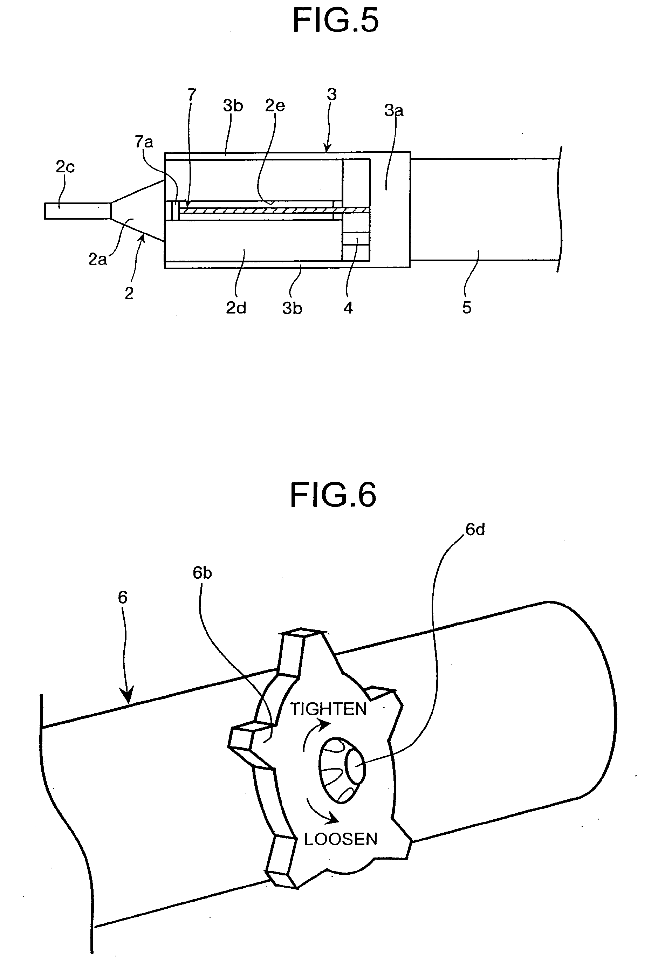

Exemplary embodiments of an ultrasonic treatment apparatus according to the present invention will be described in detail with reference to the accompanying drawings. FIG. 1 is a front view of a partial cross-section of a schematic structure of an ultrasonic treatment apparatus; FIG. 2 is a perspective view showing an ultrasonic generating unit, a supporting member, and a sheath which constitute the ultrasonic treatment apparatus shown in FIG. 1; FIG. 3 is a cross-sectional view of the ultrasonic generating unit, the supporting member, and the sheath, taken in a lengthwise direction; and FIG. 5 is a plan view of FIG. 2.

The ultrasonic treatment apparatus 1 as shown in FIG. 1 includes an ultrasonic transducer unit 2, a supporting cover 3, a wiring 4, a sheath 5, and an operation wire 7.

The ultrasonic transducer unit 2, which is disposed at the distal end of the ultrasonic treatment apparatus 1, as shown in FIGS. 1 to 5, includes a piezoelectric device 2b disposed at one end of a horn 2a, and a treatment unit 2c disposed at the other end. The rear of the horn 2a with the piezoelectric device 2b is surrounded by a cylinder 2d. The ultrasonic transducer unit 2 is provided with a wire groove 2e in a lengthwise direction on each of upper and lower sides of the cylinder 2d.

The supporting cover 3, as shown in FIGS. 1 to 5, is attached to one end of the sheath 5. The supporting cover 3 as shown in FIG. 2 is provided with two supporting arms 3b which extend frontward from a cylindrical portion 3a and whose ends are each attached to the cylinder 2d with a supporting pin 3c. The cylindrical portion 3a is provided with two pairs of wire guides 3e, one being located at upper side and the other being located at lower side. Each of the wire guides 3e is supported by a pin 3d and serves in pairs as a roller to guide the operation wire 7.

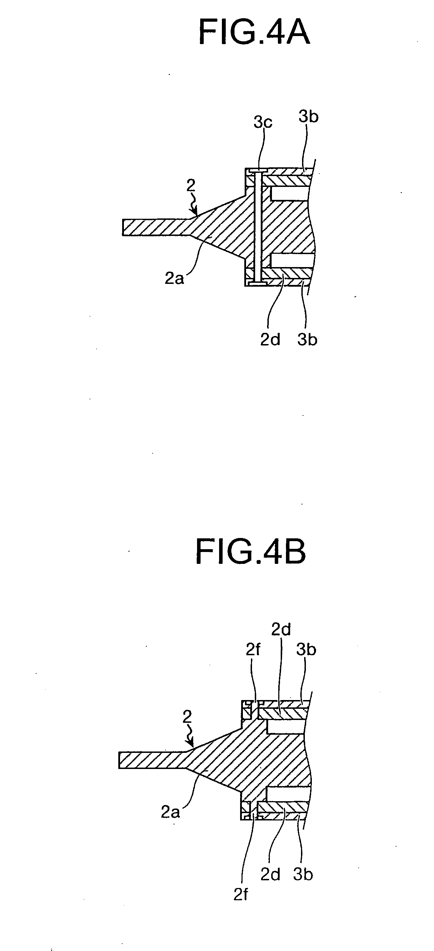

The supporting pin 3c is attached to the cylinder 2d at a position between two wire grooves 2e on the outer circumference of the cylinder 2d and at an ultrasonic vibration node. This allows suppressing the influence of the ultrasonic vibration to a minimum level, and the supporting cover 3 rotatably supports the ultrasonic transducer unit 2 about the supporting pin 3c. The pairs of wire guides 3e at upper and lower sides are located at a position shifted toward the supporting arm 3b side with respect to the extended direction of the wire groove 2e, as shown in FIG. 1. Thus, the ultrasonic transducer unit 2 can effectively rotate under a tensile force of the operation wire 7. The supporting pin 3c may be attached through the horn 2a as shown in FIG. 4A, or may be replaced with two protrusions 2f at an ultrasonic vibration node of the horn 2a to support the ultrasonic transducer unit 2 with the supporting cover 3.

The wiring 4, whose proximal portion extended from the cylinder 2d is disposed inside the sheath 5, is led to an output port 6a of the operation unit 6 to feed electrical power from an external power source to the piezoelectric device 2b, as shown in FIGS. 1 and 3. A foot switch is provided between the wiring 4 and the power source to switch on/off the piezoelectric device 2b of the ultrasonic treatment apparatus 1.

The sheath 5 is attached to the operation unit 6 at one end, and to the supporting cover 3 at the other end. The sheath 5 is fixed to the operation unit 6 and the supporting cover 3 with a laser welding or an adhesive material.

The operation unit 6 to be gripped by an operator's hand to operate the ultrasonic treatment apparatus 1 is provided with the output port 6a for the wiring 4 near the connection part with the sheath 5, as shown in FIGS. 1 and 6. As shown in FIG. 1, the operation unit 6 is provided with an operation dial 6b about midway of an outer surface of the operation unit 6, and a pulley 6c inside which is coaxial with the operation dial 6b. Turning the operation dial 6b clockwise or anti-clockwise shifts the operation wire 7 in the lengthwise direction and thus rotates the ultrasonic transducer unit 2 about the supporting pin 3c. The operation dial 6b includes a hand screw 6d which serves to hold the ultrasonic transducer unit 2 at a desired rotation position when fixed to the operation unit 6 (see FIG. 6). When turned to one direction, the hand screw 6d is loosened to release the fixation of the operation dial 6b to the operation unit 6, thereby allowing the ultrasonic transducer unit 2 to freely rotate with respect to the supporting cover 3. When turned to the other direction, the hand screw 6d is tightened to fix to the operation unit 6, thereby holding the ultrasonic transducer unit 2 against the supporting cover 3 to prevent it from rotating. The operation unit 6 is further provided with guide rollers 6e at appropriate positions inside, for guiding a movement of the operation wire 7 in the lengthwise direction depending on the turning of the operation dial 6b.

With a slack remover 8 provided near the pulley 6c inside the operation unit 6 as shown in FIG. 1, the operation wire 7 is laid inside the sheath 5 in the lengthwise direction, both ends being connected to the outside of the ultrasonic transducer unit 2 and its middle portion being wound around the pulley 6c. Both ends of the operation wire 7 are disposed in the wire grooves 2e formed on the cylinder 2d, and connected to the outside of the cylinder 2d with a wire pin 7a at an ultrasonic vibration node. This prevents the ultrasonic vibration generated in the ultrasonic transducer unit 2 from propagating to the operation wire 7, resulting in suppressing an energy loss of the ultrasonic transducer unit 2.

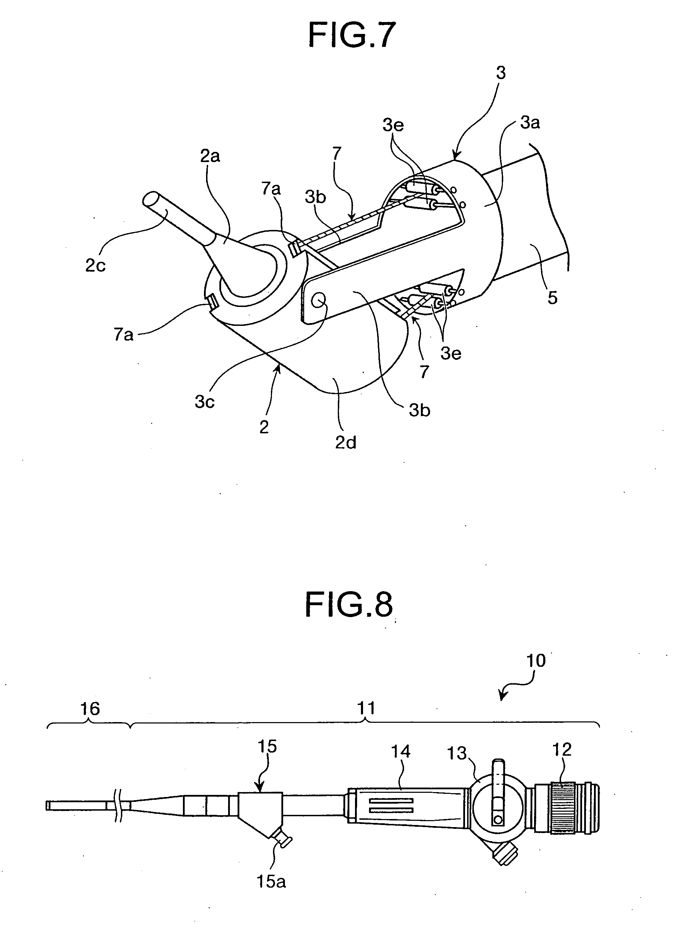

The slack remover 8 serves to absorb the slack and tension of the operation wire 7 when the operation dial 6b is turned. Inside a case 8a, a locking unit 8b having a large-diameter is stored for locking the ends of the operation wire 7. When the operation dial 6b in FIG. 1 is, for example, turned clockwise, the operation wire 7 laid at the upper side of the operation unit 6 is pulled by the pulley 6c and becomes strained, whereas the operation wire 7 laid at the lower side of the operation unit 6 becomes slack. Hence, in the ultrasonic transducer unit 2 in FIG. 7, compared with the state shown in FIG. 1, the distal end of the treatment unit 2c faces obliquely upward and accordingly the rear part of the cylinder 2d falls down. The slack remover 8 absorbs the slack and tension of the operation wire 7 when the operation dial 6b is turned, to prevent the operation wire 7 from getting tangled and allow the ultrasonic transducer unit 2 to rotate smoothly.

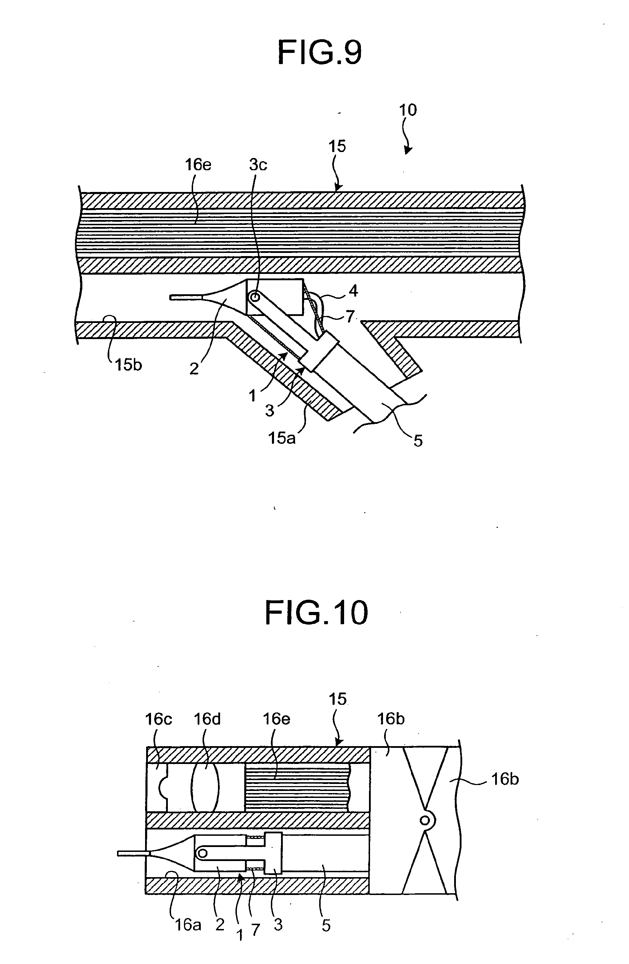

The ultrasonic treatment apparatus 1 having above described structure is, for example, used in an endoscope 10 shown in FIG. 8 as follows. Here, the endoscope 10 includes an operation unit 11 and an insert part 16 which is inserted into a body cavity. The operation unit 11 includes an eyepiece unit 12, a main body of the operation unit 13 having a hollow spherical form, a grip unit 14, and a main body of joint 15. A venting cap 15a is attached to the main body of joint 15 at a slant for inserting the ultrasonic treatment apparatus 1, and communicated with a channel 15b inside of the main body of joint 15 as shown in FIGS. 8 and 9. The insert part 16 has a channel 16a formed inside (see FIG. 10) which is communicated with a channel formed in the operation unit 11, and has a bendable structure with plural curved frames 16b which are coupled with each other as shown in FIG. 10. The insert part 16 is provided with an observation window 16c and an objective lens 16d at the distal end of the insert part 16, allowing an observation of the inside body cavity via an image guide 16e.

The ultrasonic transducer unit 2 of the ultrasonic treatment apparatus 1 is first inserted via the venting cap 15a as shown in FIG. 9. Here, the endoscope 10 has a K branch structure where the venting cap 15a is inclined to the channel 15b, which requires the fixation of the operation dial 6b of the ultrasonic treatment apparatus 1 to be released by loosening the hand screw 6d, in advance of insertion into the venting cap 15a.

When the ultrasonic transducer unit 2 of the ultrasonic treatment apparatus 1 is inserted as shown in FIG. 9, the ultrasonic transducer unit 2 freely rotates about the supporting pin 3c with respect to the supporting cover 3 under a press force given from the operation unit 6. Compared with a conventional ultrasonic treatment apparatus which does not allow the ultrasonic transducer unit to rotate, the ultrasonic treatment apparatus 1 is easily inserted into the channel 15b via the venting cap 15a as the ultrasonic transducer unit 2 rotates to adapt to the shapes of the inner wall of the venting cap 15a and the channel 15b.

The ultrasonic treatment apparatus 1 is inserted into the channel 15b via the venting cap 15a provided to the main body of joint 15, and reaches the channel 16a of the insert part 16 as shown in FIG. 10. Then, the treatment device 2c is projected from the distal end of the insert part 16 of the endoscope 10 to find a diseased part by operating the operation unit 11 as observed through the eyepiece unit 12. When the diseased position is found, the ultrasonic transducer unit 2 is directed to the diseased part by turning the operation dial 6b, and then the operation dial 6b is fixed to the operation unit 6 by turning the handle screw 6d. Thus, the ultrasonic transducer unit 2 is fixed in the direction of the diseased part, thereby allowing further insertion of the ultrasonic treatment apparatus 1 into the endoscope 10, and an execution of ultrasonic treatment with the distal end of the treatment device 2c directly applied to the diseased part.

When the ultrasonic treatment is finished, the hand screw 6d is turned in the loosening direction to release the fixation of the operation dial 6b, and the ultrasonic treatment apparatus 1 is pulled out from the endoscope 10 by holding the operation unit 6. Here, since the fixation of the operation dial 6b is released in the ultrasonic treatment apparatus 1, the ultrasonic transducer unit 2 freely rotates about the supporting pin 3c with respect to the supporting cover 3. Hence, the ultrasonic treatment apparatus 1 moves smoothly without being stuck due to an interference of the ultrasonic transducer unit 2 with the inner walls of channels 15b and 16a and the venting cap 15a when pulled out of the endoscope 10. The ultrasonic treatment apparatus 1 can be inserted and removed easily into and from the channels 15b and 16a of the endoscope 10.

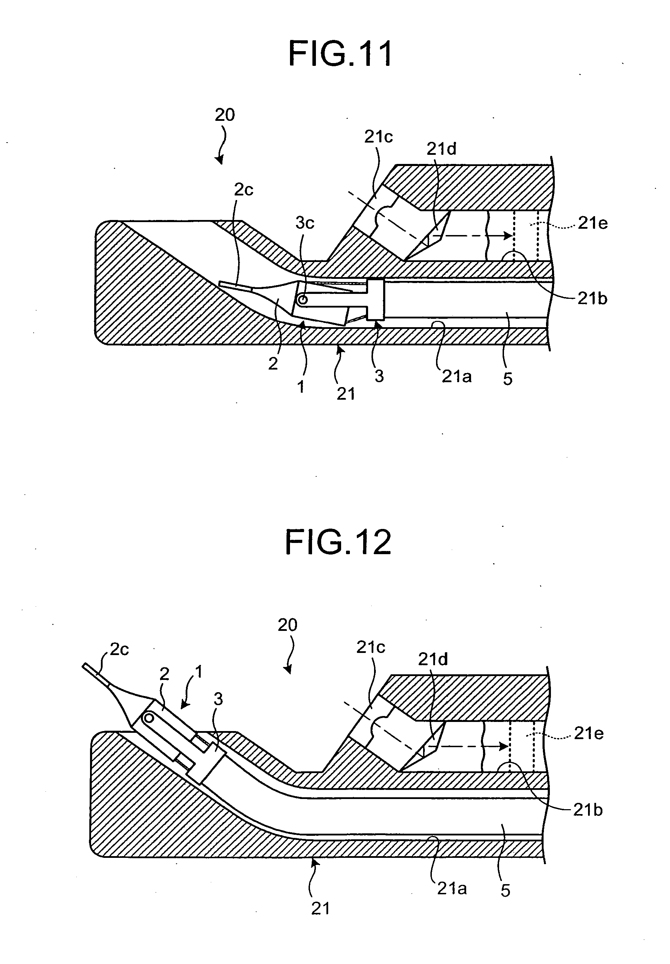

Moreover, when the ultrasonic treatment apparatus 1 is an oblique-viewing endoscope 20 as shown in FIG. 11 which has an inclined channel 21a at the distal end of the insert part 21 to be inserted into the body cavity, it is also easy to insert and remove the ultrasonic treatment apparatus 1. As shown in FIG. 11, in the oblique-viewing endoscope 20, the end of the channel 21a into which the ultrasonic treatment apparatus 1 is inserted and the end of a channel 21b where an optical system is arranged are inclined upward. The channel 21b is, as shown in FIG. 11, provided with an observation window 21c, an observation optical system 21d, and an image pick-up device 21e located at an imaging position of the observation optical system 21d.

After the hand screw 6d is loosened to release the fixation of the operation dial 6b in advance as described, the ultrasonic treatment apparatus 1 is inserted into the channel 21a of the oblique-viewing endoscope 20. The ultrasonic treatment apparatus 1 is inserted into the channel 21a as the ultrasonic transducer unit 2 supported by the supporting cover 3 rotates about the supporting pin 3c to move in accordance with the shape of the channel 21a. Then, the ultrasonic treatment apparatus 1 easily goes through even around a part with obliquely upward slope at the end of the channel 21a as shown in FIG. 11. Accordingly, the treatment device 2c of the ultrasonic transducer unit 2 of the ultrasonic treatment apparatus 1 is projected from the end of the channel 21a as shown in FIG. 12.

Under this condition, the diseased part is found by operating the oblique-viewing endoscope 20 as observed through the eyepiece unit, the operation dial 6b is turned to direct the ultrasonic transducer unit 2 to the diseased part, and the hand screw 6d is turned to fix the operation dial 6b. Thus, the ultrasonic transducer unit 2 is fixed in the direction of the diseased part, thereby allowing further insertion of the ultrasonic treatment apparatus 1 into the channel 21a, and an execution of ultrasonic treatment with the distal end of the treatment unit 2c directly applied to the diseased part. When the ultrasonic treatment is finished, the hand screw 6d is loosened to release the fixation of the operation dial 6b, and the ultrasonic treatment apparatus 1 can be easily pulled out of the endoscope 20 by holding the operation unit 6.

In the ultrasonic treatment apparatus 1 according to the present invention as described above, the ultrasonic transducer unit 2 is rotatably attached to the supporting cover 3. As a result, the ultrasonic treatment apparatus 1 can be inserted into and removed easily from the channel of the endoscope and have an advantage in usability. Moreover, the ultrasonic transducer unit 2 of the ultrasonic treatment apparatus 1 is supported with the supporting cover 3 at an ultrasonic vibration node, thereby allowing suppressing an influence of the ultrasonic vibration on the supporting cover 3 to a minimum level, and an energy loss of the ultrasonic transducer unit 2 as little as possible.

### INDUSTRIAL APPLICABILITY

The ultrasonic treatment apparatus according to the present invention as described above is advantageous when inserted into and removing from a channel of an endoscope for ultrasonic treatment, specifically as an ultrasonic treatment apparatus used in an endoscope whose channel has a branch or a slant.

## Claims

1. An ultrasonic treatment apparatus for giving ultrasonic treatment to a desired part in a body cavity with an ultrasonic generating unit projected from a distal end of art endoscope introduced into the body cavity, comprising:
a supporting member which rotatably supports the ultrasonic generating unit at an ultrasonic vibration node; and
an operation wire which operates to rotate the ultrasonic generating unit.

2. The ultrasonic treatment apparatus according to claim 1, wherein the supporting member is attached to one end of a sheath which is provided with an operation unit at the other end.

3. The ultrasonic treatment apparatus according to claim 2, wherein the operation unit includes an operation dial for operating the operation wire.

4. The ultrasonic treatment apparatus according to claim 3, wherein the operation wire is laid inside the sheath, both ends being connected to an outside of the ultrasonic generating unit at the ultrasonic vibration node and a middle portion being wound around a winding member provided with the operation dial.

5. The ultrasonic treatment apparatus according to claim 3 or 4, wherein the operation dial includes a fixing unit which fixes an operation position and holds the ultrasonic generating unit at a desired position.
